(19) Europäisches Patentamt
European Patent Office
Office européen des brevets

(11) Numéro de publication : **0 152 324**
**B1**

# (12) FASCICULE DE BREVET EUROPÉEN

(45) Date de publication du fascicule du brevet : **12.11.86**

(51) Int. Cl.⁴ : **C 07 C 11/21**, C 07 C 1/30

(21) Numéro de dépôt : **85400123.7**

(22) Date de dépôt : **25.01.85**

(54) **Nouveau tétraène, sa préparation et son emploi.**

(30) Priorité : **26.01.84 FR 8401198**

(43) Date de publication de la demande : **21.08.85 Bulletin 85/34**

(45) Mention de la délivrance du brevet : **12.11.86 Bulletin 86/46**

(84) Etats contractants désignés : **AT BE CH DE FR GB IT LI LU NL SE**

(56) Documents cités : **NEANT**

(73) Titulaire : **RHONE-POULENC SANTE**
**Les Miroirs 18 Avenue d'Alsace**
**F-92400 Courbevoie Cedex (FR)**

(72) Inventeur : **Mignani, Gérard**
**2 avenue des Frères Lumière**
**F-69008 Lyon (FR)**
Inventeur : **Morel, Didier**
**3 rue Jean Jaurès**
**F-91700 Villiers-sur-Orge (FR)**

(74) Mandataire : **Pilard, Jacques et al**
**RHONE-POULENC RECHERCHES Service Brevets**
**Pharma 25, Quai Paul Doumer**
**F-92408 Courbevoie Cedex (FR)**

**EP 0 152 324 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne le méthyl-2 méthylène-6 octatriène-1,3,7 de formule :

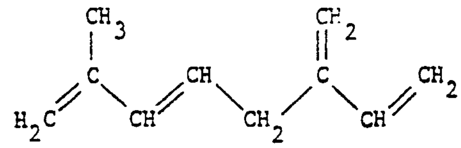

(I)

sa préparation à partir du chloro-3 méthyl-2 méthylène-6 octadiène-1,7 et son emploi.

Il est connu, en particulier par travaux de J. Tsuji Tetrahedron Letters, *24,* 2075-2078 (1978) et Ann. New York Acad. Sciences, 260 (1980) ou ceux de Chaci, J. of Molecular Catalysis, *19,* 189-200 (1983) d'effectuer des réactions d'élimination en utilisant comme catalyseur des dérivés du palladium (II) ou du palladium (0) en présence d'un ligand. Cependant ces réactions ne sont pas toujours sélectives et conduisent le plus souvent à l'obtention de produits isomères difficilement séparables.

Il a maintenant été trouvé que le méthyl-2 méthylène-6 octatriène-1,3,7 de formule (I) peut être obtenu par déhydrochloration du chloro-3 méthyl-2 méthylène-6 octadiène-1,7 (ou chloro-3 myrcène) dans des conditions sélectives qui ne conduisent pas à l'obtention de l'isomère diméthyl-2,6 octatétraène-1,3,5,7 (ou cosmène).

Selon la présente invention, la déhydrohalogénation du chloro-3 myrcène peut être effectuée en présence d'un catalyseur à base de palladium bivalent ou de palladium zérovalent associé à un ligand et d'une base minérale et éventuellement d'un sel d'ammonium quaternaire.

Comme dérivés du palladium bivalent qui conviennent particulièrement bien peuvent être cités $PdCl_2$, $Pd(OCOCH_3)_2$, $Pd(NO_3)_2$, Pd(acétylacétonato)$_2$, $PdCl_2(PPh_3)_2$, $PdCl_2(PhCN)_2$, $PdCl_2(CH_3CN)_2$, $(C_3H_5PdCl)_2$, $(C_3H_5Pd\ OCOCH_3)_2$, $(C_3H_5)_2Pd$.

Comme dérivés du palladium zérovalent conviennent particulièrement bien les dérivés du type $PdL_4$ dans lesquels L représente un ligand choisi parmi les phosphines, les diphosphines, les phosphites, les arsines et les stibines et Pd (dibenzylidène acétone)$_2$.

Comme ligands associés aux dérivés du palladium, conviennent particulièrement bien les dérivés du phosphore, de l'arsenic et de l'antimoine tels que les phosphines, les arsines et stibines.

Généralement, on utilise une quantité molaire de catalyseur comprise entre 0,01 et 0,1 par mole de chloro-3 myrcène mis en œuvre.

La base minérale nécessaire à la mise en œuvre du procédé selon l'invention est généralement choisie parmi les hydroxydes de métaux alcalins, tels que la soude ou la potasse, sous forme d'une poudre finement broyée. Généralement on utilise une quantité équimoléculaire de base par rapport au chloro-3 myrcène utilisé.

Dans le but de favoriser la vitesse de réaction, il est particulièrement avantageux d'ajouter au mélange réactionnel un sel d'ammonium quaternaire tel que le chlorure, le bromure ou le sufate de tétrabutylammonium. Il est possible également d'utiliser l'hydroxyde de tétrabutylammonium. On utilise généralement de 0,05 à 0,2 mole de sel d'ammonium quaternaire par mole de chloro-3 myrcène.

La réaction est mise en œuvre dans un solvant organique anhydre choisi parmi les éthers comme l'éther diéthylique ou le tétrahydrofuranne et les hydrocarbures aromatiques comme le benzène ou le toluène.

Afin d'éviter la polymérisation du produit obtenu, la réaction est, de préférence, conduite à une température inférieure à 25 °C.

Le produit de formule (I) est isolé du mélange réactionnel selon les techniques habituelles après élimination du catalyseur.

Le produit de formule (I) est particulièrement utile comme intermédiaire dans la synthèse de la vitamine A.

Ainsi, soumis à l'action d'un composé à groupement méthylène actif tel que l'acétylacétate de méthyle dans les conditions décrites dans le brevet européen 44 771, le produit de formule (I) conduit au produit de formule :

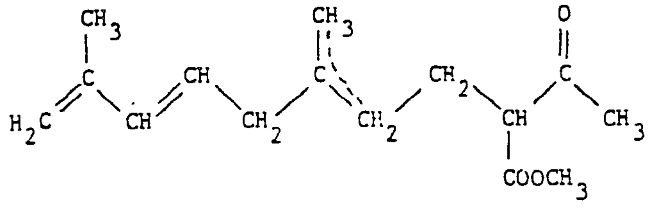

(II)

qui, après décarboxylation et hydrogénation fournit la tétrahydrogéranylacétone ou hexahydropseudoionone.

Le produit de formule (II), après décarboxylation, peut être isomérisé en pseudoionone par chauffage en présence d'un catalyseur à base de rhodium.

Les exemples suivants, donnés à titre non limitatif, montrent comment l'invention peut être mise en pratique.

Exemple 1

Dans un ballon de 250 $cm^3$, on introduit, sous atmosphère d'argon, 11,98 g (299,5 m · moles) de soude finement broyée, 3,59 g (15,1 m · moles) de triphénylphosphine, 2,76 g (9,9 m · moles) de chlorure de tétrabutylammonium et 1,09 g (3 m · moles) de $[(C_3H_5)_2PdCl]_2$. Après avoir purgé trois fois l'appareil à l'argon, on introduit 100 $cm^3$ de tétrahydrofuranne anhydre et 53,26 g (312 m · moles) de chloro-3 myrcène. On maintient pendant 20 heures à une température voisine de 20 °C. Après avoir ajouté 100 $cm^3$ d'eau au mélange réactionnel on extrait par 3 fois 50 $cm^3$ de pentane. Les couches organiques réunies sont séchées sur sulfate de magnésium. Après filtration et évaporation du solvant, on obtient 51,94 g d'une huile jaune. Par distillation sous pression réduite, on obtient 27,2 g d'une huile incolore contenant 93,3 % de méthyl-2 méthylène-6 octatriène-1,3,7 (P.E. = 54 °C sous 9 mm de mercure ; 1,2 kPa).

L'analyse par chromatographie en phase gazeuse avec un étalon interne permet de déterminer que :

— le taux de transformation du chloro-3 myrcène est de 94,6 %,

— le rendement en méthyl-2 méthylène-6 octatriène-1,3,7 est de 86,3 % par rapport au chloro-3 myrcène transformé.

La structure du méthyl-2 méthylène-6 octatriène-1,3,7 est confirmée par le spectre infra-rouge, le spectre de résonance magnétique nucléaire du proton, le spectre ultra-violet et le spectre de masse.

Le chloro-3 myrcène, utilisé comme produit de départ, peut être préparé de la manière suivante :

On utilise un réacteur de 4 litres muni d'une agitation mécanique, d'un thermomètre, d'un dispositif de barbotage de gaz, d'une arrivée de pentane (réglée par une pompe), d'une colonne de distillation dont la sortie est reliée à un ballon de 5 litres contenant de la soude 1,5 N, que l'on fait circuler au moyen d'une pompe de haut en bas d'une colonne garnie d'anneaux de Raschig surmontant le ballon (afin d'éliminer l'acide chlorhydrique entraîné par le pentane), et relié lui-même à un ballon dans lequel le pentane est condensé.

Dans le réacteur, on introduit, sous atmosphère d'argon, 840 g (6,176 moles) de myrcène pur et 2 litres de pentane. On chauffe le ballon au moyen d'un bain à 45-55 °C. La température du mélange réactionnel est de 35 °C. On fait alors passer un mélange gazeux de chlore et d'argon, le rapport des débits de chlore et d'argon étant 7/12. On élimine par distillation le pentane et l'acide chlorhydrique formé tout en ajoutant simultanément du pentane afin de maintenir un volume constant. La vitesse d'addition du chlore est de 167 g/heure et celle du pentane de 2 litres/heure. Au bout de 2 heures 40 minutes, on a introduit 440 g de chlore (6,19 moles) et 5 litres de pentane. Le volume de pentane recueilli est de 5,5 litres. Après la fin de l'addition du chlore, le pentane contenu dans le réacteur est éliminé par distillation à 40 °C sous 5,3 kPa. On obtient ainsi un résidu pesant 1 080 g qui est distillé rapidement entre 34 et 60 °C sous 0,08 kPa. Le distillat obtenu (1 026 g) titre 86,9 % en chloro-3 myrcène. Le rendement est de 84,6 % par rapport au myrcène mis en œuvre.

Le dosage du myrcène dans les distillats légers montre que le taux de conversion est de 96,4 %. Le rendement est de 87,8 % par rapport au myrcène ayant réagi.

Exemple 2

Dans un ballon de 250 $cm^3$, on introduit, sous atmosphère d'argon, 13,0 g (325 m · moles) de soude finement broyée, 1 g (0,74 m · mole) de $Pd/P(C_6H_5)_3/_4$ et 2,86 g (10,3 m · moles) de chlorure de tétrabutylammonium. Après avoir purgé 3 fois à l'argon, on ajoute 100 $cm^3$ de tétrahydrofuranne anhydre et 47 g (275,4 m · moles) de chloro-3 myrcène. On agite pendant 48 heures à une température voisine de 20 °C. On ajoute 100 $cm^3$ d'eau puis on extrait par 3 fois 20 $cm^3$ de pentane. Les phases organiques réunies sont séchées sur sulfate de magnésium. Après filtration et évaporation du solvant, on obtient 26,31 g d'une huile jaune. Après distillation sous pression réduite, on obtient 14,21 g d'une huile incolore contenant par dosage par chromatographie en phase vapeur, 89,6 % de méthyl-2 méthylène-6 octatriène-1,3,7.

L'analyse par chromatographie en phase gazeuse avec étalon interne permet de déterminer que :

— le taux de transformation du chloro-3 myrcène est de 94,5 %,

— le rendement en méthyl-2 méthylène-6 octatriène-1,3,7 est de 53,2 % par rapport au chloro-3 myrcène transformé.

Exemple 3

Dans un ballon de 500 $cm^3$, on introduit, sous atmosphère d'argon, 5,32 g (50,2 m · moles) de carbonate de sodium, 10,65 g (17,04 m · moles) de tri(m · sulfophényl)phosphine, sel de sodium (TPPTS Na) et 0,38 g de [Rh Cl (cyclooctadiène-1,5)$]_2$ soit 1,54 m · mole de rhodium. Après avoir purgé 3 fois à

l'argon, on ajoute 100 $cm^3$ d'un mélange eau-méthanol (75-25 en volumes), 80 $cm^3$ (742 m · moles) d'acétylacétate de méthyle et 33,6 g (248,5 m · moles) de méthyl-2 méthylène-6 octatriène-1,3,7 dont la pureté est de 92,5 % (d'après l'analyse par chromatographie en phase gazeuse). On agite pendant 24 heures à 60 °C. Après décantation, la phase aqueuse est extraite par 100 $cm^3$ d'éther. Les phases organiques réunies sont lavées par 50 $cm^3$ d'eau puis séchées sur sulfate de magnésium. Après filtration et évaporation du solvant, on obtient 60,88 g d'une huile rouge foncé.

L'analyse par chromatographie en phase gazeuse montre que le taux de transformation du méthyl-2 méthylène-6 octatriène-1,3,7 est de 98 %.

Les analyses spectrales (RMN, infra-rouge, masse) montrent que le produit obtenu est constitué d'un mélange 55/45 des produits de formule :

$$CH_2{=}C(CH_3){-}CH{=}CH{-}CH_2{-}C(CH_3){=}CH{-}CH_2{-}CH(COOCH_3){-}CO{-}CH_3 \text{ et}$$
$$CH_2{=}C(CH_3){-}CH{=}CH{-}CH_2{-}C({=}CH_2){-}CH_2{-}CH_2{-}CH(COOCH_3){-}CO{-}CH_3$$

A 60,88 g de produit ainsi obtenu en solution dans 60 $cm^3$ de méthanol on ajoute 60 $cm^3$ d'une solution de soude 4N. On agite fortement la solution à une température de 45 °C pendant 3 heures.

Le mélange réactionnel est neutralisé par addition d'acide sulfurique à 50 %. Après extraction par 100 $cm^3$ d'éther, on obtient 57,95 g d'une huile jaune clair. Après distillation à 118-120 °C sous pression réduite (3 mm de mercure ; 0,4 kPa), on obtient 15,99 g d'un mélange des produits isomères de formule :

$$CH_2{=}C(CH_3){-}CH{=}CH{-}CH_2{-}C(CH_3){=}CH{-}CH_2CH_2{-}CO{-}CH_3$$
$$CH_2{=}C(CH_3){-}CH{=}CH{-}CH_2{-}C({=}CH_2){-}CH_2{-}CH_2{-}CH_2{-}CO{-}CH_3$$

La structure des produits obtenus est confirmée par le spectre infra-rouge, le spectre de masse et le spectre de résonance magnétique nucléaire du proton.

## Exemple 4

Dans un autoclave en acier inoxydable de 125 $cm^3$, on introduit 1 g du mélange obtenu à l'exemple 3 (soit 5,2 m · moles), 10 $cm^3$ de méthanol et 0,3 g de palladium sur noir à 10 % de palladium. Après avoir purgé à l'azote on établit une pression d'hydrogène de 50 bars. On agite pendant 12 heures à 50 °C. Après filtration et évaporation du solvant, on obtient, avec un rendement de 67 %, 0,7 g d'hexahydropseudoionone (3,5 m · moles) de formule :

$$(CH_3)_2CH{-}CH_2{-}CH_2{-}CH_2{-}CH(CH_3){-}CH_2{-}CH_2{-}CH_2{-}CO{-}CH_3$$

dont la structure est confirmée par le spectre de masse et le spectre de résonance magnétique nucléaire du $^{13}C$.

## Exemple 5

Dans un ballon de 50 $cm^3$, on introduit, sous atmosphère d'argon, 10 $cm^3$ de toluène sec, 610 mg du mélange obtenu à l'exemple 3 et 25,7 mg de Rh CO $(TPP)_3$. On laisse réagir pendant 48 heures au reflux.

L'analyse par chromatographie en phase gazeuse montre la présence du mélange des pseudoionones cis et trans de formule :

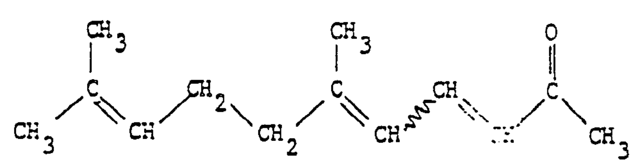

**Revendications** (pour les Etats contractants : BE, CH, DE, FR, GB, IT, LI, LU, NL, SE)

1. Le méthyl-2 méthylène-6 octatriène-1,3,7 de formule :

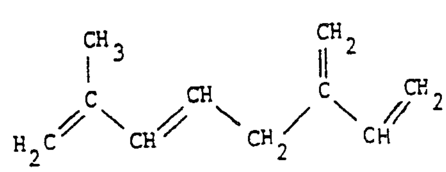

2. Procédé de préparation du produit selon la revendication 1, caractérisé en ce que l'on effectue la

déhydrochloration du chloro-3 méthyl-2 méthylène-6 octadiène-1,7 de formule :

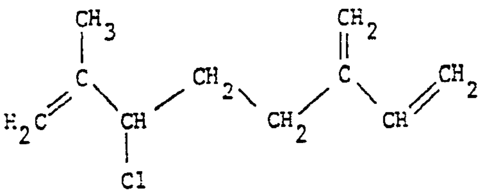

en présence d'un catalyseur à base de palladium bivalent ou de palladium (0) associé à un ligand et d'une base minérale et éventuellement d'un sel d'ammonium quaternaire.

3. Procédé selon la revendication 2, caractérisé en ce que le catalyseur est choisi parmi $PdCl_2$, $Pd(OCOCH_3)_2$, $Pd(NO_3)_2$, Pd(acétylacétonato)$_2$, $PdCl_2[P(C_6H_5)_3]_2$, $PdCl_2(C_6H_5—CN)_2$, $PdCl_2(CH_3—CN)_2[C_3H_5PdCl]_2$,$[C_3H_5PdOCOCH_3]_2$, $(C_3H_5)_2Pd$, les dérivés Pd $L_4$ dans lesquels L représente un ligand choisi parmi les phosphines, les diphosphines, les phosphites, les arsines et les stibines, et Pd(dibenzylidène acétone)$_2$.

4. Procédé selon la revendication 2, caractérisé en ce que la base est choisie parmi les hydroxydes de métaux alcalins.

5. Procédé selon la revendication 2, caractérisé en ce que l'on opère dans un solvant organique anhydre choisi parmi les éthers et les hydrocarbures aromatiques.

6. Procédé selon la revendication 2, caractérisé en ce que l'on opère à une température inférieure à 25 °C.

**Revendications** (pour l'Etat contractant AT)

1. Procédé de préparation du méthyl-2 méthylène-6 octatriène-1,3,7 de formule :

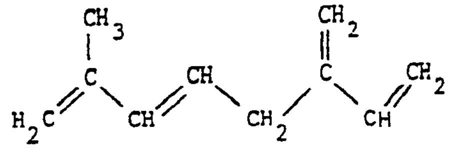

caractérisé en ce que l'on effectue la déhydrochloration du chloro-3 méthyl-2 méthylène-6 octadiène-1,7 de formule :

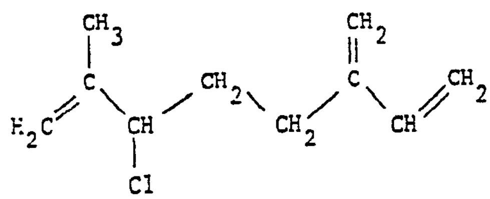

en présence d'un catalyseur à base de palladium bivalent ou de palladium (0) associé à un ligand et d'une base minérale et éventuellement d'un sel d'ammonium quaternaire.

2. Procédé selon la revendication 1 caractérisé en ce que le catalyseur est choisi parmi $PdCl_2$, $Pd(OCOCH_3)_2$, $Pd(NO_3)_2$, Pd(acétylacétonato)$_2$, $PdCl_2[P(C_6H_5)_3]_2$, $PdCl_2(C_6H_5—CH)_2$, $PdCl_2(CH_3—CN)_2$ $[C_3H_5PdCl]_2$, $[C_3H_5PdOCOCH_3]_2$, $(C_3H_5)_2Pd$, les dérivés Pd $L_4$ dans lesquels L représente un ligand choisi parmi les phosphines, les diphosphines, les phosphites, les arsines et les stibines, et Pd(dibenzylidène acétone)$_2$.

3. Procédé selon la revendication 1, caractérisé en ce que la base est choisie parmi les hydroxydes de métaux alcalins.

4. Procédé selon la revendication 1, caractérisé en ce que l'on opère dans un solvant organique anhydre choisi parmi les éthers et les hydrocarbures aromatiques.

5. Procédé selon la revendication 1, caractérisé en ce que l'on opère à une température inférieure à 25 °C.

**Claims** (for the Contracting States : BE, CH, DE, FR, GB, IT, LI, LU, NL, SE)

1. 2-Methyl-6-methylene-1,3,7-octatriene of formula

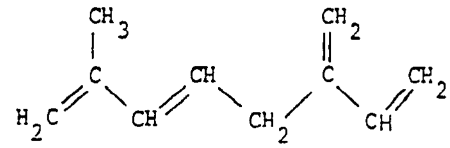

2. A process for the preparation of the product according to claim 1, in which dehydrochlorination of 3-chloro-2-methyl-6-methylene-1,7-octadiene of formula :

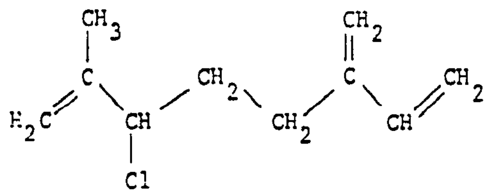

is carried out in the presence of a catalyst based on divalent palladium or on palladium (0) associated with a ligand and of an inorganic base and, if appropriate, a quaternary ammonium salt.

3. A process according to claim 2, in which the catalyst is chosen from $PdCl_2$, $Pd(OCOCH_3)_2$, $Pd(NO_3)_2$, Pd(acetylacetonato)$_2$, $PdCl_2[P(C_6h_5)_3]_2$, $PdCl_2(C_6H_5—CN)_2$, $PdCl_2(CH_3—CN)_2$, $[C_3H_5PdCl]_2$, $[C_3H_5PdOCOCH_3]_2$, $(C_3H_5)_2Pd$, the Pd $L_4$ derivatives in which L denotes a ligand chosen from phosphines, diphosphines, phosphites, arsines and stibines, and Pd(dibenzylideneacetone)$_2$.

4. A process according to claim 2, in which the base is chosen from alkali metal hydroxides.

5. A process according to claim 2, in which the operation is performed in an anhydrous organic solvent chosen from ethers and aromatic hydrocarbons.

6. A process according to claim 2, in which the operations is performed at a temperature below 25 °C.

**Claims** (for the Contracting State AT)

1. A process for the preparation of 2-methyl-6-methylene-1,3,7 octadiene of formula :

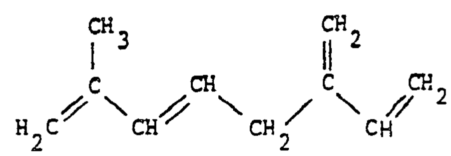

in which dehydrochlorination of 3-chloro-2-methyl-6-methylene-1,7-octadiene of formula :

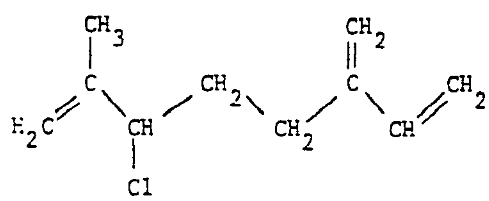

is carried out in the presence of a catalyst based on divalent palladium or palladium or on palladium (0) associated with a ligand and of an inorganic base and, if appropriate, a quaternary ammonium salt.

2. A process according to claim 1, in which the catalyst is chosen from $PdCl_2$, $Pd(OCOCH_3)_2$, $Pd(NO_3)_2$, Pd(acetylacetonato)$_2$, $PdCl_2[P(C_6H_5)_3]_2$, $PdCl_2(C_6H_5—CN)_2$, $PdCl_2(CH_3—CN)_2$, $[C_3H_5PdCl]_2$, $[C_3H_5PdOCOCH_3]_2$, $(C_3H_5)_2Pd$, the Pd $L_4$ derivatives in which L denotes a ligand chosen from phosphines, diphosphines, phosphites, arsines and stibines, and Pd(dibenzylideneacetone)$_2$.

3. A process according to claim 1, in which the base is chosen from alkali metal hydroxides.

4. A process according to claim 1, in which the operation is performed in an anhydrous organic solvent chosen from ethers and aromatic hydrocarbons.

5. A process according to claim 1, in which the operation is performed at a temperature below 25 °C.

**Patentansprüche** (für die Vertragsstaaten : BE, CH, DE, FR, GB, IT, LI, LU, NL, SE)

1. 2-Methyl-6-methylen-1,3,7-octatrien der Formel

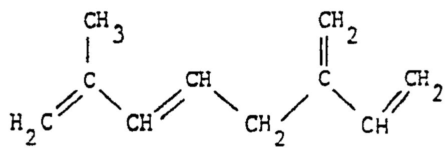

2. Verfahren zur Herstellung des Produkts gemäß Anspruch 1, dadurch gekennzeichnet, daß man die Dehydrochlorierung des 3-Chlor-2-methyl-6-methylen-1,7-octadiens der Formel

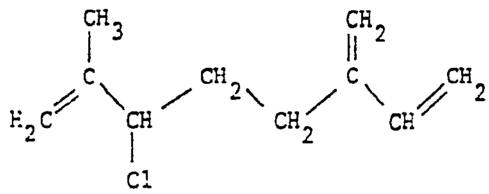

in Gegenwart eines Katalysators auf Basis von zweiwertigem Palladium oder Palladium (0), assoziiert an einen Liganden, und einer Mineralbase und gegebenenfalls eines quaternären Ammoniumsalzes durchführt.

3. Verfahren gemäß Anspruch 2, dadurch gekennzeichnet, daß der Katalysator ausgewählt ist unter $PdCl_2$, $Pd(OCOCH_3)_2$, $Pd(NO_3)_2$, $Pd(Acetylacetonato)_2$, $PdCl_2[P(C_6H_5)_3]_2$, $PdCl_2(C_6H_5{-}CN)_2$, $PdCl_2(CH_3{-}CN)_2$, $[C_3H_5PdCl]_2$, $[C_3H_5PdOCOCH_3]_2$, $(C_3H_5)_2Pd$, den Pd $L_4$-Derivaten, worin L einen Liganden, ausgewählt unter den Phosphinen, den Diphosphinen, den Phosphiten, Arsinen und Stibinen, und $Pd(Dibenzylidenaceton)_2$, darstellt.

4. Verfahren gemäß Anspruch 2, dadurch gekennzeichnet, daß die Base ausgewählt ist unter den Alkalimetallhydroxyden.

5. Verfahren gemäß Anspruch 2, dadurch gekennzeichnet, daß man in einem wasserfreien organischen Lösungsmittel, ausgewählt unter den Ethern und aromatischen Kohlenwasserstoffen, arbeitet.

6. Verfahren gemäß Anspruch 2, dadurch gekennzeichnet, daß man bei einer Temperatur unterhalb 25 °C arbeitet.

**Patentansprüche** (für den Vertragsstaat AT)

1. Verfahren zur Herstellung von 2-Methyl-6-methylen-1,3,7-octatrien der Formel :

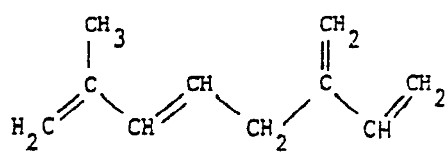

dadurch gekennzeichnet, daß man die Dehydrochlorierung von 3-Chlor-2-methyl-6-methylen-1,7-octadien der Formel :

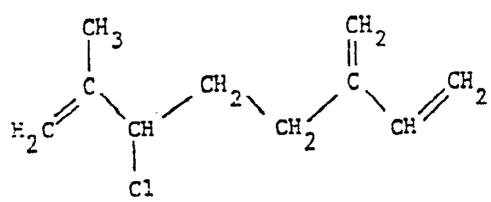

in Gegenwart eines Katalysators auf Basis von zweiwertigemm Palladium oder Palladium (0) in Kombination mit einem Liganden und einer anorganischen Base und gegebenenfalls eines quaternären Ammoniumsalzes ausführt.

2. Verfahren nach Anspruch 1, dadurch gekennzeichnet, daß der Katalysator ausgewählt ist aus PdCl-2, $Pd(OCOCH_3)_2$, $Pd(NO_3)_2$, $Pd(acetylacetonat)_2$, $PdCl_2[P(C_6H_5)_3]_2$, $PdCl_2(C_6H_5{-}CN)_2$, $PdCl_2(CH_3{-}CN)_2$, $[C_3H_5PdCl]_2$, $[C_3H_5PdOCOCH_3]_2$, $(C_3H_5)_2Pd$, den Pd $L_4$-Derivaten, worin L einen Liganden darstellt, ausgewählt aus den Phosphinen, den Diphosphinen, den Phosphiten, den Arsinen und den Stibinen, und $Pd(dibenzylidenaceton)_2$.

3. Verfahren nach Anspruch 1, dadurch gekennzeichnet, daß die Base ausgewählt ist aus den Alkalimetallhydroxiden.

4. Verfahren nach Anspruch 1, dadurch gekennzeichnet, daß man in einem wasserfreien organischen Lösungsmittel, ausgewählt aus den Äthern und den aromatischen Kohlenwasserstoffen, arbeitet.

5. Verfahren nach Anspruch 1, dadurch gekennzeichnet, daß man bei einer Temperatur unterhalb 25 °C arbeitet.